# EUROPÄISCHE PATENTSCHRIFT

(11) **EP 0 538 754 B1**
(45) Veröffentlichungstag und Bekanntmachung des Hinweises auf die Patenterteilung: **14.01.1998**
(21) Anmeldenummer: 92117775.4
(22) Anmeldetag: 17.10.1992
(51) Int. Cl.: A61K 39/395, G01N 33/577

(54) **Verwendung von Antikörper enthaltenden Präparationen zur Immunsuppression**
Use of antibody-containing preparations for immunosuppression
Utilisation de préparations contenant des anticorps pour l'immunosuppression

(30) Priorität: 23.10.1991 DE 4134982
(43) Veröffentlichungstag der Anmeldung: 28.04.1993
(73) Patentinhaber: Forschungszentrum Karlsruhe GmbH, 76133 Karlsruhe (DE); Deutsches Krebsforschungszentrum Heidelberg, 69009 Heidelberg (DE); BOEHRINGER INGELHEIM INTERNATIONAL GmbH, 55218 Ingelheim am Rhein (DE)
(72) Erfinder: Zöller, Margot, Dr., W-6900 Heidelberg (FR); Herrlich, Peter, Prof. Dr., W-7500 Karlsruhe 41 (FR); Ponta, Helmut, Prof. Dr., W-7515 Linkenheim-Hochstetten (FR)
(74) Vertreter: Laudien, Dieter, Dr.

(56) Entgegenhaltungen:
- EP-A- 0 303 463
- EP-A- 0 501 233
- WO-A-91/17248
- INTERNATIONAL JOURNAL OF CANCER Bd. 46, Nr. 5 , 15. November 1990 , GENF, SCHWEIZ Seiten 919 - 927 S. REBER ET AL. 'Retardation of metastatic tumor growth after immunization with metastasis-specific monoclonal antibodies.'
- IMMUNOBIOLOGY Bd. 183, Nr. 3-4 , Oktober 1991 , STUTTGART, DEUTSCHLAND Seite 221 S SEITER ET AL. 'Expression of variant CD44 transfers the metastatic phenotype and antibodies against variant CD44 inhibit metastatic spread in lymphatic tissue.'
- CELL Bd. 65, Nr. 1 , 5. April 1991 , CAMBRIDGE MA, VSA Seiten 13 - 24 U. GÜNTHERT ET AL. 'A new variant of glycoprotein CD44 confers metastatic potential to rat carcinoma cells.'
- JOURNAL OF CELLULAR BIOCHEMISTRY Bd. SUPPL.15, Nr. E , 1991 , NEW YORK, VSA Seite 185 B. ROTHMAN ET AL. 'T cell activation via CD3 is inhibited by a monoclonal antibody to CD44.'
- KERNFORSCHUNGZENTRUM KARLSRUHE NACHRICHTEN Bd. 23, Nr. 4 , 1991 , KARLSRUHE, DEUTSCHLAND Seiten 177 - 180 H. PONTA 'vCD44: A membrane glycoprotein is necessary and sufficient for metastasis formation.'

## Beschreibung

Gegenstand der vorliegenden Erfindung ist die Verwendung von gegen Varianten des Glykoproteins CD44 (vCD44) gerichtete Antikörper, insbesondere monoklonale Antikörper, zur Immunsuppression bei Säugetieren und beim Menschen.

Bei CD44 handelt es sich um ein auf der Zelloberfläche lokalisiertes Glykoprotein, welches ursprünglich als "lymphocyte homing receptor" beschrieben wurde und soll bei der Adhäsion von Lymphocyten an bestimmten mucösen Endothelzellen von Venen (Peyer's patch oder Peyer-Plagues bzw. Folliculi lymphatici aggregati) bzw. postkapillaren Venen der Lymphknoten beteiligt sein (S.T. JALKANEN et al., Eur. J. Immunol. 16, 1195-1202, 1986; R.L. CAMP et al., J. Exp. Med. 173, 763-766, 1991). Darüber hinaus wird dem CD44 Glykoprotein eine Beteiligung bei der Reifung und Aktivierung von Lymphozyten zugeschrieben bzw. einen die erhöhte Migrationsfähigkeit aller Lymphoblasten (mit)bedingenden Effekt (z.B. R.L. CAMP et al., 1991, loc. cit; S. HUET et al., J. Immunol. 143, 798-801, 1989) und soll eine Rolle als Ankerstelle für andere Adhäsionsmoleküle spielen (Y. SHIMIZU et al., J. Immunol. 143, 2457-2463, 1989). Bis heute sind jedoch nicht alle diese Funktionen von CD44 eindeutig geklärt.

Kürzlich konnte bei Ratten-Tumorzellen, die über das lymphatische System metastasieren (BSp73-Zellen eines spontanen Ratten Pankreas-Adenokarzinoms), festgestellt werden, daß diese Zellen Varianten von CD44 (vCD44) exprimieren und für die Verbreitung ("trafficking") von Tumorzellen verantwortlich sind. An anderen Tumorzellinien konnten ebenfalls diese Verhältnisse nachgewiesen werden.

Es konnte gezeigt werden, daß dieses vCD44 Glykoprotein einen a priori nicht metastasierenden Tumor Metastasefähigkeiten verleiht, während das Standard-Typ CD44 (sCD44) dazu nicht in der Lage ist. Somit kann heute davon ausgegangen werden, daß vCD44 gegenüber sCD44 ein metastasespezifisches Protein ist, welches Tumoren die Fähigkeit verleiht, über die Lymphbahnen zu metastasieren (U. GÜNTHERT et al., Cell 65, 13-24, 1991).

Die weitere Aufklärung des vCD44 Glykoproteins der Ratte bis hin zur endgültigen Charakterisierung der DNA- und Aminosäuresequenz gelang U. GÜNTHERT et al., 1991, loc. cit., anhand des BSp73-Rattenzellsystems, welches aus zwei morphologisch bzw. phenotypisch verschiedenen syngenen Zellvarianten besteht: einer nichtmetastasierenden Variante AS (BSp73AS) und einer metastasierenden Variante ASML (BSp73ASML) (S. MATZKU et al., Cancer Research 49, 1294-1299, 1989).

Zu diesem Zweck wurden monoklonale Antikörper (mAbs) hergestellt, die die antigene Determinante auf der metastasierenden Variante BSp73ASML erkennen.

Sowohl aus dem Primärtumor (subcutaner nicht-metastasierender Knoten bestehend aus BSp73AS-Zellen) als auch einer Metastase davon (BSp73ASML-Zellen, welche in Lymphknoten und Lunge metastasieren) wurden Zellinien gewonnen. Es wurden mAbs hergestellt, die gegen die Membranproteine von BSp73ASML-Zellen gerichtet sind (S. MATZKU et al., 1989, loc. cit.). Einer von diesen mAbs, der nur Epitope auf BSp73ASML-, nicht jedoch solche auf BSp73AS-Zellen oder anderen nicht-tumorogenen Zellen erkennt, wurde dazu benutzt, um eine E. coli cDNA-Expressionsbibliothek, hergestellt aus poly(A)⁺RNA von BSp73ASML-Zellen und einem geeigneten Vektorsystem, zu durchsuchen (screening). Auf diesem Weg konnte ein Klon identifiziert werden (pMeta-1) der die vollständige cDNA mit einer Länge von 3207 bp enthält und welche für eine zusätzliche Domäne von 162 Aminosäuren codiert. Diese Domäne ist weder in sCD44-Zellen noch in anderen nicht-metastasierenden Tumorzellen zu finden und enthält die mAb spezifische Epitop-codierende Region. Anhand von mRNA Präparationen aus Zellen verschiedener Geweben und damit durchgeführter mRNA:DNA Hybridisierungen mit unterschiedlichen von den cDNA Klonen erhaltenen DNA Proben war festzustellen, daß vCD44 eine Spleiß-Variante von sCD44 darstellt und daß die Expression der vCD44 RNAs mit der Entstehung von Metastasen einhergeht. Damit steht fest, daß die durch die 486 bp lange Insertion codierte zusätzliche extrazelluläre Domäne (Aminosäuren 224 bis 385 in pMeta-1) der metastasenrelevante Anteil des Oberflächenglykoproteins vCD44 ist.

Das metastatische Tumorwachstum (Adenokarzinom der Ratte) konnte nach Immunisierung mit monoklonalen Antikörpern, die das vorgenannte Epitop erkennen bzw. die spezifisch mit diesen extrazellulären Bereich von vCD44 reagieren, unterdrückt werden (S. REBER et al., Int. J. Cancer 46, 919-927, 1990).

Die Identifizierung dieser varianten extrazellulären Domäne in der Ratte (pMeta-1 bzw. rMeta-1) ermöglicht auch die dazu äquivalenten menschlichen Nukleotid- und Aminosäure-Sequenzen aufzuklären:

Mit einer von der DNA der vCD44 Domäne der Ratte abgeleiteten cDNA Sonde können durch Hybridisierung, gegebenenfalls unter stringenten Bedingungen, mit genomischer DNA aus verschiedenen speziesspezifischen Zellinien (beispielsweise Mensch, Ratte, Maus) homologe Bereiche in diesen DNAs gefunden werden. Eine solche Probe kann anschließend für die Hybridisierung gegen RNAs aus verschiedenen humanen Zellinien, insbesondere Tumorzellinien, beispielsweise großzellige Lungenkarzinome, Melanome, Colonkarzinome, Brusttumore, Keratinocyten, benutzt werden. Auf diese Weise kann ohne Weiteres eine geeignete Humanzellinie, beispielsweise die eines großzelligen Lungenkarzinoms, aufgefunden werden, welche zu der cDNA Probe der Ratte homologe Sequenzen enthält. Über PCR (polymerase chain reaction, Polymerase-Kettenreaktion), ein bekanntes in vitro - Verfahren zur selektiven Anreicherung von DNA-Bereichen definierter Länge und definierter Sequenz aus einem Gemisch von DNA-Molekülen unter Verwendung einer DNA-Polymerase und einem geeigneten Primer, können cDNAs von RNA Präparationen aus verschiedenen Humanzellinien, beispielsweise Zellen von großzelligen Lungenkarzinomen, Melanomen, Kolonkarzinomen, immortalisierte Keratinocyten, erhalten werden, welche für sCD44 und vCD44 codieren.

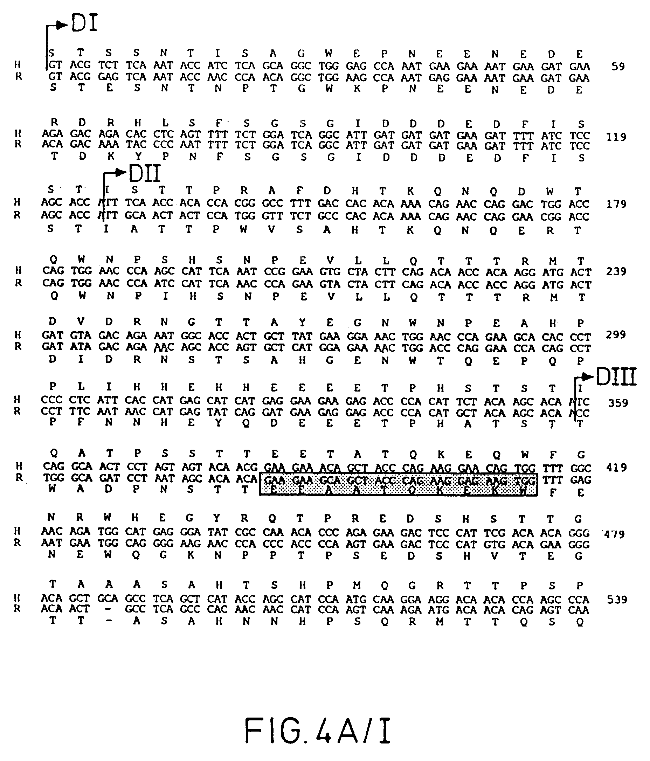

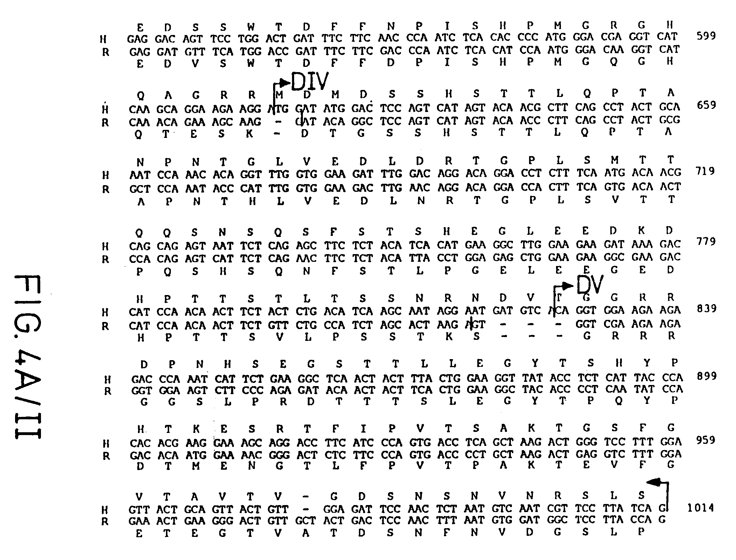

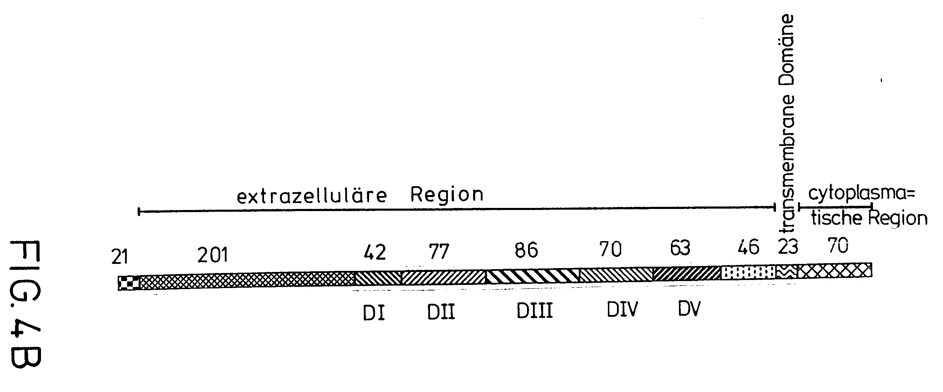

Eine derartige, über PCR erhaltene cDNA kann in einen geeigneten Klonierungsvektor nach bekannten Methoden einligiert und, nach entsprechender Kultivierung der damit transformierten Wirtszellen, vorzugsweise Bakterien, beispielsweise E.coli, anschließend sequenziert werden. Auf diese Weise erhält man aus den unterschiedlichsten Humanzellen, ausgewählt aus allen möglichen Tumorzellen bzw. Zellinien, insbesondere aus solchen, die Metastasierungseigenschaften aufweisen, DNA-Sequenzen, die entweder für ein normales Human sCD44 Glykoprotein bekannter Größenordnung im Bereich von 350 Aminosäuren codieren (I. STAMENKOVIC et al., Cell 56, 1057-1062, 1989), welches eine extrazelluläre Domäne im Bereich von 85 kDa enthält (S. JALKANEN et al., J. Cell Biol. 105, 983-990, 1987) oder die für ein variantes Human vCD44 Glykoprotein codieren, welches eine zusätzliche extrazelluläre Domäne unterschiedlicher Länge, insbesondere zwischen 850 bp und 1.5 kb, beispielsweise 1014 bp (oder 338 Aminosäuren), umfassen kann, und die innerhalb der für das normale sCD44 Glykoprotein codierenden DNA-Sequenz inseriert ist, beispielsweise zwischen den Positionen der Nukleotide 782 und 783 (Fig. 4). Eine derartige DNA Sequenz kann in mehreren, beispielsweise fünf, Domänen vorliegen, welche unterschiedliche Exons repräsentieren und die sowohl in verschiedenen Tierzellinien (beispielsweise der Ratte oder Maus) als auch Humanzellinien gefunden werden können.

Die in Fig. 4A beispielhaft dargestellte cDNA, welche homolog ist zum längsten erhaltenen varianten Teil aus humanen Tumorzellinien, wurde aus der Rattentumorzellinie BSpASML über PCR isoliert. Ein direkter Vergleich der davon abgeleiteten Aminosäuresequenz mit derjenigen des aus einer menschlichen Tumorzellinie erhaltenen Human-Klons zeigt, daß Domäne I 83%, Domäne II 83%, Domäne III 71%, Domäne IV 82% und Domäne V 66% Homologie zur Ratten DNA Sequenz aufweist. Insgesamt ergibt sich daraus, daß ca. 76% der Human-Sequenzen in den varianten Bereichen gegenüber der Ratten-Sequenz konserviert sind. Die korrespondierenden Rattensequenzen, welche dem Tumor metastatisches Potential verleihen (U. GÜNTHERT et al., 1991, loc. cit.), umfassen die Aminosäuren 258 bis 420 und werden durch die Domänen II und III codiert.

Dem Fachmann war damit klar, daß poly- oder monoklonale Antikörper, die spezifisch diese Epitope, d.h. diesen zusätzlichen extrazellulären Bereich auf verschiedenen Spleiß-Varianten von vCD44, erkennen und damit reagieren, und die durch Radioisotope markiert und/oder mit cytocidal oder cytotoxisch wirkenden Stoffen konjugiert sein können, zur diagnostischen und therapeutischen Verwendung bei einem metastasierenden Tumorgeschehen des Menschen zu verwenden sind.

Der oben kurz dargestellte Sachverhalt ist Gegenstand der internationalen Anmeldung WO 91/17248 (Stand der Technik gemäß Art. 54(3) EPÜ)

Die europäische Patentanmeldung EP-A 0 501 233 (Stand der Technik gemäß Art. 54(3) EPÜ) offenbart Antikörper, welche immunologisch reaktiv sind gegen das Standard-Typ CD44 (sCD44) T-Zelloberflächen Antigen und welche OKT-3 induzierte T-Zell Proliferation inhibiert. Insbesondere beschreibt die EP-A 0 501 233 den monoklonalen Antikörper mAB 212.3, welcher für die Immunsuppression in einem Säugetier Anwendung finden soll.

Von ROTHMAN B. L. et al.; J. Cellular Biochemistry 15 (Suppl.) Nr. E, 185, Zusammenfassung 0422, 1991, wird ein gegen normales, Standard-Typ CD44 (sCD44) gerichteter anti-CD44 monoklonaler Antikörper (m Ab 212.3) beschrieben, welcher vollständig die CD3-Rezeptor vermittelte PBMC Proliferation bzw. T-Zell Aktivierung inhibierte.

Überraschenderweise wurde nun gefunden, daß Antikörper, insbesondere monoklonale Antikörper, die mit den verschiedenen, Metastasen-spezifischen Varianten von CD44 (vCD44) reagieren, immunsuppressive Wirkung entfalten; diese neue Eigenschaft war in keinster Weise aufgrund der oben geschilderten Sachverhalte zu erwarten.

Gegenstand der Erfindung ist daher die Verwendung von Antikörpern, insbesondere von monoklonalen Antikörpern, die mit Metastasen-spezifischen Varianten von CD44 (vCD44) eines Säugetieres reagieren, zur Herstellung von Präparationen für die Erzeugung einer transienten oder andauernden Immunsuppression in Säugetieren und im Mensch.

Es ist an dieser Stelle hervorzuheben, daß somit jedes beliebige variante CD44 (vCD44) Glykoprotein tierischer oder menschlicher Herkunft und die sie codierenden Nukleinsäuren (DNAs und RNAs), welche als Insert innerhalb des für sCD44 kodierenden Genbereichs vorliegen, dem Fachmann nunmehr zugänglich sind und es im Rahmen seines durchschnittlichen Fachkönnens liegt, diese Proteine und die diese Proteine kodierenden Nukleinsäuren dazu zu benutzen, um beliebige Antikörper, insbesondere monoklonale Antikörper, Fragmente und Derivate davon, für die erfindungsgemäße Verwendung herzustellen und zu benutzen.

Unter dem Begriff vCD44 oder, synonym dafür, variante extrazelluläre Domänen oder Bereiche von CD44 bzw. sCD44 sind im Rahmen der vorliegenden Erfindung, wenn von Nukleinsäuren die Rede ist, jede für ein oder mehrere vCD44 Proteine oder Domänen codierende RNA, DNA oder Transkripte davon gemeint, einschließlich solche, die durch Mutationen, beispielsweise durch Deletionen, Insertionen, Substitutionen, Inversionen, Transitionen, Transversionen verändert sind und solche, die mit der in Fig. 4A dargestellten DNA Sequenz unter den bekannten konventionellen Bedingungen hybridisieren und deren komplementäre, kodierende Stränge für ein Protein kodieren, welches Tumoren Metastaseeigenschaften verleiht, unabhängig davon, ob die Herstellung und Isolierung dieser Nukleinsäuren konventionell über Zellkulturen, oder über DNA-Rekombination, über synthetische oder semisynthetische Verfahren erfolgt.

Unter dem Begriff vCD44 oder, synonym dafür, variante extrazelluläre Domänen oder Bereiche von CD44 bzw. sCD44 sind, wenn von einem entsprechenden Oberflächenprotein die Rede ist, im Rahmen der vorliegenden Erfindung alle jene vom Tier oder vom Menschen abstammenden Glykoproteine zu verstehen, die, unabhängig von ihrer Herstellung oder Isolierung über konventionelle Zellkulturen oder über DNA Rekombination oder über synthetische oder semisynthetische Verfahren, als zusätzlicher Anteil innerhalb von sCD44 vorliegen und einem Tumor Metastaseeigenschaften verleihen.

Unter dem Begriff Antikörper sind mono- oder polyvalente Antikörper und poly- und monoklonale Antikörper zu verstehen, aber auch solche, die Fragmente davon darstellen und Derivate davon, einschließlich der F(ab')₂, Fab' und Fab Fragmente, aber auch chimäre Antikörper oder Hybridantikörper mit mindestens zwei Antigen- bzw. Epitopbindungsstellen, oder bispezifische rekombinante Antikörper (beispielsweise Quadrome, Triome), Interspecies-Hybridantikörper, Anti-idiotypische Antikörper und solche davon, die chemisch modifiziert wurden und als Derivate dieser Antikörper zu verstehen sind und die entweder über die bekannten konventionellen Verfahren der Antikörpergewinnung oder über DNA-Rekombination, via Hybridomatechniken oder Antikörper-Engineering oder synthetisch oder semisynthetisch nach an sich bekannter Weise herstellbar sind und Neutralisierung- oder Bindungseigenschaften hinsichtlich des oben beschriebenen und definierten vCD44 aufweisen. Aus der vielfältigen Literatur sei nur beispielhaft hingewiesen auf Arbeiten von KÖHLER, G. & MILSTEIN, C., Nature 256, 495-497, 1975; BIOCCA, S. et al., EMBO J. 9, 101-108, 1990; BIRD, R.E. et al., Science 242, 423-426, 1988; BOSS, M.A. et al., Nucl. Acids Res. 12, 3791-3806, 1984; BOULIANNE, G.L. et al., Nature 312, 643-646, 1984; BUKOVSKY, J. & KENNETT, R.H., Hybridoma 6, 219-228, 1987, DIANO, M. et al., Anal. Biochem. 166, 223-229, 1987; HUSTON J.S. et al., Proc. Natl. Acad. Sci. USA 85, 5879-5883, 1988; JONES, P.T. et al., Nature 321, 522-525, 1986; LANGONE, J.J. & VUNAKIS, H.V. (Hrsg.), Methods Enzymol. 121, Academic Press. London, 1987; MORRISON, S. et al., Proc. Natl. Acad. Sci. USA 81, 6851-6855, 1984; OI, V.T. & MORRISON, S.L., BioTechniques 4, 214-221, 1986; RIECHMANN, L. et al., Nature 332, 323-327, 1988; TRAMONTANO, A. et al., Proc. Natl. Acad. Sci. USA 83, 6736-6740, 1986; WOOD, C.R. et al., Nature 314, 446-449, 1985.

Hinsichtlich der Herstellung von polyklonalen Antikörpern gegen Epitope von vCD44 stehen eine Anzahl Verfahren zur Verfügung. Es können z.B. für diesen Zweck in an sich bekannter Weise verschiedene Tiere durch Injektion mit vCD44, welches natürlichen Ursprungs, über DNA-Rekombination oder synthetisch hergestellt sein kann, oder Fragmenten davon, immunisiert werden und aus den danach gewonnenen Seren die gewünschten polyklonalen Antikörper nach bekannten Methoden gewonnen und gereinigt werden. Als Alternative können auch intakte Zellen benutzt werden. Verschiedene Adjuvantien zur Erhöhung der Immunantwort auf die vCD44-Gabe können, abhängig von dem für die Immunisierung ausgewählten Tier, ebenfalls verwendet werden - beispielsweise Freund's Adjuvant, Mineralgele wie z.B. Aluminiumhydroxid, oberflächenaktive Substanzen wie z.B. Polyanionen, Peptide, Ölemulsionen, Hemocyanine, Dinitrophenol oder Lysolecithin.

Die für die erfindungsgemäße Verwendung bevorzugten monoklonalen Antikörper gegen ein Epitop von vCD44 können durch jede beliebige Technik erhalten werden, die für die Herstellung von Antikörpern über Kultivierung von Zellinien zur Verfügung stehen. Zu derartigen bekannten Techniken zählen z.B. die von KÖHLER, G. & MILSTEIN, C., 1975, loc. cit., oder TAGGART & SAMLOFF, Science 219, 1228-1230, 1983, beschriebenen Verfahren mit Hybridomazellen oder solche mit humanen B Zell Hybridomen (KOZBOR et al. Immunology Today 4, 72-79, 1983). Chimäre Antikörper gegen vCD44 können beispielsweise aus einer Maus Antigen-Bindungsdomäne und humanen konstanten Regionen zusammengesetzt sein (MORRISON, et al., Proc. Natl. Acad. Sci. USA 81, 6851-6855, 1984; TAKEDA, et al., Nature 314, 452-454, 1985).

Die Antikörper können nach bekannten Methoden gereinigt werden, beispielsweise über Immunoabsorptions- oder Immunoaffinitätschromatographie, über HPLC (High Performance Liquid Chromatography) oder Kombinationen davon. Antikörper Fragmente, welche den Idiotyp des Moleküls enthalten, können gleichermaßen nach bekannten Verfahren hergestellt werden. Beispielsweise können F(ab')₂ Fragmente durch Pepsin Verdauung des vollständigen poly- oder monoklonalen Antikörpers erhalten werden. Fab' Fragmente können erhalten werden,indem beispielsweise die Disulfidbrücken des betreffenden F(ab')₂ Fragments reduziert werden und Fab Fragmente können geschaffen werden beispielsweise durch Behandlung der Antikörpermoleküle mit Papain und einem Reduktionsmittel.

Zur Identifzierung und Selektion von Antikörpern, Fragmenten oder Derivaten davon, die mit einem Epitop von vCD44 reagieren, kann jedes bekannte Verfahren verwendet werden. Beispielsweise dadurch, daß diese nach entsprechender Markierung dedektierbar sind, wenn sie an isoliertes oder gereinigtes vCD44 gebunden haben oder über Immunpräzipitation des z.B. über Polyacrylamidgele gereinigten vCD44, oder dadurch, daß Antikörper gegen vCD44 mit anderen vCD44-Antikörpern um das Binden an vCD44 konkurrieren.

Auf weitere Einzelheiten betreffend die generelle Verwendung von monoklonalen Antikörpern zur Immunsuppression und bei Autoimmunkrankheiten, von Hybridantikörpern für therapeutische Zwecke und über DNA Rekombination hergestellte Antikörper sei verwiesen auf Progress in Allergy Vol. 45, "Monoclonal Antibody Therapy", 1988 und auf die Arbeit von SEAMAN, W.E. et al., Ann. Rev. Med. 39, 231-241, 1988.

Die zusätzliche Domäne in vCD44 sowohl im Tier (z.B. Ratte) als auch diejenige des Menschen ist hinsichtlich der Stoffparameter (DNA- und Aminosäuresequenz, Lokalisation innerhalb des kompletten für CD44 codierenden Gens) und ihrer Herstellung vollständig offenbart, sodaß es dem Fachmann anhand dieser Offenbarung ermöglicht wird, beliebige Antikörper bzw. monklonale Antikörper im Sinne der oben angegebenen Definitionen für jedes auf dieser zusätzlichen extrazellulären Domäne von vCD44 lokalisierte Epitop herzustellen und sie erfindungsgemäß zu benutzen, sodaß ihre Verwendung nicht auf bestimmte spezifische Antikörper oder die sie produzierenden Hybridzellinien beschränkt ist. Beispielsweise ist das Epitop, das der mAb 1.1ASML erkennt, durch die Aminosäuresequenz E-E-A-A-T-Q-K-E-K-W bzw. Glu Glu Ala Ala Thr Gln Lys Glu Lys Trp exakt definiert (Fig. 4A).

Die erfindungsgemäße Verwendung von derartigen Antikörpern, Fragmenten und Derivaten davon ist im Stand der Technik nicht beschrieben.

Aufgrund ihrer immunsuppressiven Wirkung sind die bezeichneten Antikörper bzw. die sie enthaltenen Präparationen zur Verhinderung und Behandlung von Krankheiten und Zuständen geeignet, die einer vorrübergehenden oder dauerhaften Verringerung oder Unterdrückung einer Immunantwort bedürfen. Insbesondere erstreckt sich ihr Einsatz zur Suppression der Aktivierung der Proliferation von Lymphocyten oder cytotoxischen T-Zellen und/oder Immunocyten, beispielsweise zur Verhinderung oder Behandlung von Autoimmunkrankheiten, wie z.B. Erkrankungen des rheumatischen Formenkreises, Multiple Sclerose, Psoriasis, atopische Dermatitis, oder zur Verhinderung der Abstoßung von transplantierten Geweben oder Organen wie z.B. Niere, Herz, Lunge, Knochenmark, Milz, Cutis oder Cornea, bei unerwünschten Reaktionen während oder nach Transfusionen, von allergischen Erkrankungen, besonders solchen, die den gastro-intestinalen Trakt betreffen und dort endzündlich manifest werden können, oder von endzündlichen, proliferativen und hyperproliferativen Erkrankungen und cutanen Manifestationen immunologisch bedingter Erkrankungen, wie z.B. ekzematöse Dermatitiden, Urticaria, Vasculitiden, Sclerodermie.

Die erfindungsgemäße Verwendung beruht auf der unerwarteten Beobachtung, daß ein gegen den varianten Anteil von CD44 (vCD44) gerichteter Antikörper die T-Zell abhängige und T-Zell unabhängige Immunantwort in vivo und in vitro stark verminderte. Dadurch, daß durch ein oder mehrere der oben beschriebenen Antikörper, Fragmente oder Derivate davon erreicht wird, daß die Wirkung von vCD44, von der anzunehmen ist, daß sie nicht nur für die humurale Immunantwort sondern auch für die Aktivität der Proliferation und cytotoxischen T-Zellen erforderlich ist, neutralisiert wird, wird durch die erfindungsgemäße Verwendung dieser Antikörper eine gezielte und - abhängig von der Menge, Dauer und Beschaffenheit der zu applizierenden Antikörperpräparation - hinsichtlich der Intensität und der Andauer steuerbare Suppression einer Immunantwort möglich. Somit ist insbesondere der klinische Einsatz dieser Antikörper für die oben näher beschriebenen Störungen, Erkrankungen und Zustände, d.h. wenn eine Immunsuppression in einem tierischen oder menschlichen Organismus erwünscht ist, besonders vorteilhaft.

Abhängig von der Art und Ursache der zu behandelnden Erkrankung oder Störung oder des zu beeinflussenden Zustands in einem tierischen oder menschlichen Körper kann es wünschenswert sein, die Antikörperpräparation systemisch, lokal oder topisch an das oder in das betreffende Gewebe oder Organ zu applizieren. Eine systemische Wirkungsweise ist beispielsweise dann erwünscht, wenn unterschiedliche Organe oder Organsysteme behandlungsbedürftig sind, wie z.B. bei systemischen Autoimmunkrankheiten oder Allergien oder bei Transplantationen fremder, größerer Organe oder Gewebe. Demgegenüber wäre eine lokale Wirkung in Betracht zu ziehen, wenn nur örtliche Manifestationen eines immunologischen Geschehens zu beeinflussen sind, wie beispielsweise bei kleinflächigen Transplantationen von Cutis, und Cornea oder bei lokaler Dermatitis.

Die betreffenden Antikörper können über jede dem Fachmann bekannte enterale oder parenterale Applikationsroute verabreicht werden. Für die systemische Applikation bietet sich beispielsweise die intravenöse, intravaskuläre, intramuskuläre, intraarterielle, intraperitoneale, orale oder intrathecale Route an. Eine eher lokale Verabreichung kann beispielsweise subcutan, intracutan, intrakardial, intralobär, intramedullär, intrapulmonal oder in oder an das zu behandelnde Gewebe (Binde-, Knochen-, Muskel-, Nerven-, Epithel- oder Knochengewebe) erfolgen. Abhängig von der zu erzielenden Dauer und Stärke der immunsuppressiven Wirkung können die Antikörperpräparationen einmal oder mehrmals, auch intermittierend, pro Tag über mehrere Tage, Wochen oder Monate und unterschiedlichen Dosen verabfolgt werden. Zur Herstellung einer für die genannten Applikationen geeignete Antikörperpräparation können die dem Fachmann bekannten injizierbaren, physiologisch verträglichen Lösungen in steriler Form verwendet werden. Zur Herstellung einer gebrauchsfertigen Lösung zur parenteralen Injektion oder Infusion stehen die bekannten wässrigen isotonischen Lösungen, beispielsweise Saline oder eine entsprechende Plasmaproteinlösung ohne Gammaglobulin, zur Verfügung. Die Präparation kann aber auch in Form eines Lyophilisates bzw. Trockenpräparates vorliegen, welches mit einer der bekannten injizierbaren Lösungen unmittelbar vor dem Gebrauch unter sterilen Bedingungen rekonstituiert werden kann, z.B. als kit-of-parts. Die endgültige Herstellung einer erfindungsgemäß zu verwendenden Antikörperpräparation für die Injektion, Infusion oder Perfusion erfolgt durch Vermischen von nach bekannten Methoden gereinigten Antikörpern gemäß oben angegebener Definitionen mit einem der genannten physiologisch verträglichen Lösungen, welche gegebenenfalls supplementiert sein können mit bekannten Träger- oder Hilfsstoffen (z.B. Serumalbumine, Dextrose, Natriumbisulfit, EDTA).

Die Menge der zu verabreichenden Antikörper hängt ab von der Art und Schwere der zu behandelnden Krankheit oder Störung oder des zu beeinflussenden Zustands und des betreffenden Patienten (Tier oder Mensch). Auszugehen ist jedoch von einer zu verwendenden Dosierung von 1 bis 1000 mg, vorzugsweise 5-200 mg des betreffenden Antikörpers pro Dosiseinheit, wie sie auch für andere Antikörper bzw. monoklonale Antikörper üblich ist, wobei zwischen 0.01 bis 20 mg / Tag und 0.1 bis 100 mg / kg Körpergewicht / Tag auch über längere Zeit (Tage, Wochen, Monate) gegeben werden kann, um die gewünschten Effekte zu erreichen - je nachdem, wie intensiv und über welchen Zeitraum eine Immunsuppression erzielt werden soll.

### LEGENDEN ZU DEN FIGUREN:

Fig. 1: Kreuzhybridisierung von vCD44 Sequenzen zwischen Ratte, Maus und Mensch. Bahn 1= Ratte (Leberzellen), Bahn 2= Maus (L-Zellen), Bahn 3= Mensch (Mammakarzinomzellinie T47D)
Fig. 2: Expression von vCD44 und sCD44 Sequenzen in menschlichen Tumorzellinien. Bahn 1= LCLC103, Bahn 2= LCLC97, Bahn 3= CH3LC, Bahn 4= EPLC32M1, Bahn 5= SCLC24, Bahn 6= SCLC18. Bahn 7 entspricht der Rattenzellinie BSp73ASML und Bahn 8 der Rattenzellinie BSp73AS. GAPDH= Glycerinaldehydphosphatdehydrogenase zur relativen Mengenbestimmung der aufgetragenen RNA-Mengen.
Fig. 3: Agaroselgelelektrophoretische Auftrennung von über PCR Amplifikation erhaltenen, zu den in verschiedenen humanen Zellinien exprimierten CD44 RNAs komplementären cDNAs. Bahn 1= SW620, Bahn 2= MeWo, Bahn 3= HT29, Bahn 4= LCLC97, Bahn 5= HPKII, Bahn 6= Längenmarker, Boehringer Mannheim, Nr. 7.
Fig. 4A: DNA- und Aminosäuresequenz der vCD44 Region der Zellinie LCLC97 des Menschen. Zum Vergleich ist die DNA- und Aminosäuresequenz der Ratten Tumorzellinie BSpASML, welche von der erhaltenen DNA abgeleitet wurde, angegeben. Die Pfeile geben die Grenzen der fünf Exons bzw. Domänen D I bis D V an. H= Mensch, R= Ratte. Das Epitop, welches der monoklonale Antikörper 1.1ASML erkennt, ist gekennzeichnet durch 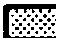 .
Fig. 4B: Schematische Wiedergabe der fünf Exons (Domänen) D I bis D V innerhalb der extrazellulären Region von LCLC97. Zahlenangaben = Anzahl Aminosäuren
Fig. 5: Einfluß von Anti-vCD44 (1.1ASML) auf die allogene Aktivierung von T-Lymphocyten (gemessen als ³H-Thymidin Einbau nach Stimulierung=CPM). I=Milzzellen, II=Lymphknotenzellen, jeweils von DA Ratten. Immunisierung der DA Ratten mit BDX (Lymphocyten, mit 3000R bestrahlt).
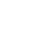 = Immunisierung mit BDX, stimuliert mit BDX in vitro
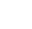 = Immunisierung mit BDX in Gegenwart von 1.1ASML, stimuliert mit BDX in vitro
□ = Immunisierung und Stimulierung mit BDX in Gegenwart von 1.1ASML
Fig. 6: Einfluß von Anti-vCD44 (1.1ASML) auf die Aktivierung von cytotoxischen T-Zellen, primäre CTL. Immunisierung von DA Ratten mit BDX (Lymphocyten, mit 3000R bestrahlt).
□ = Immunisierung mit BDX
■ = Immunisierung mit BDX in Gegenwart von 1.1ASML
E:T = Verhältnis von Effektorzellen (E, Milzzellen) zu Zielzellen (T, ⁵¹Cr-markierte BDX Lymphoblasten).

Die folgenden Beispiele sollen die Erfindung näher erläutern.

### Beispiel 1: Charakterisierung von vCD44 des Menschen

### 1.1 Identifizierung von homologen vCD44 Sequenzen zwischen Ratte, Maus und Mensch

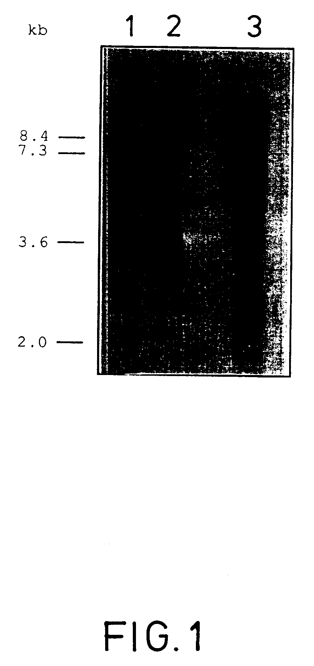

Aus Rattenleber, L-Zellen der Maus und der Mammakarzinomzellinie T47D des Menschen (ATCC Nr. HTB133) wurde jeweils die genomische DNA nach bekannter Methode (T. MANIATIS, E.F. FRITSCH, J. SAMBROOK in: Molecular Cloning, a laboratory manual, 1982, Cold Spring Harbor) isoliert. Jeweils 10 µg davon wurden nach Standardverfahren mit EcoRI vollständig verdaut und für die anschließende Kreuzhybridisierung mit einer Hybridisierungsprobe, bestehend aus dem cDNA Anteil der Positionen 941-1108 von pMeta-1 des varianten CD44 Bereichs der Ratte (U. GÜNTHERT et al., Cell 65, 13-24, 1991), für die DNA:DNA Southern-Hybridisierung nach bekannter Methode präpariert und filterfixiert. Die Hybridisierung erfolgte über Nacht bei 65°C in 6xSSC. Die Filter wurden anschließend drei mal für jeweils 30 Minuten bei 65°C in Waschpuffer (2xSSC, 0.1% SDS) gewaschen und schließlich einmal in 0.5xSSC, 0.1% SDS. Es wurden bei Mensch, Ratte und Maus DNA Fragmente gefunden, die eindeutig homolog zueinander sind (Fig. 1).

### 1.2 Expression von vCD44 Sequenzen in Tumorzellen des Menschen

Aus den bekannten Lungenkarzinomzellinien SCLC18, SCLC24, EPLC32M1, CK3LC, LCLC97, LCLC103, für die sowohl die Kulturbedingungen als auch ihre Eigenschaften beschrieben sind (G. BEPLER et al., J. Cancer Res. and Clin. Oncol. 113, 31-40, 1987; G. BEPLER et al., Differentiation 37, 158-171, 1988; H.-H.

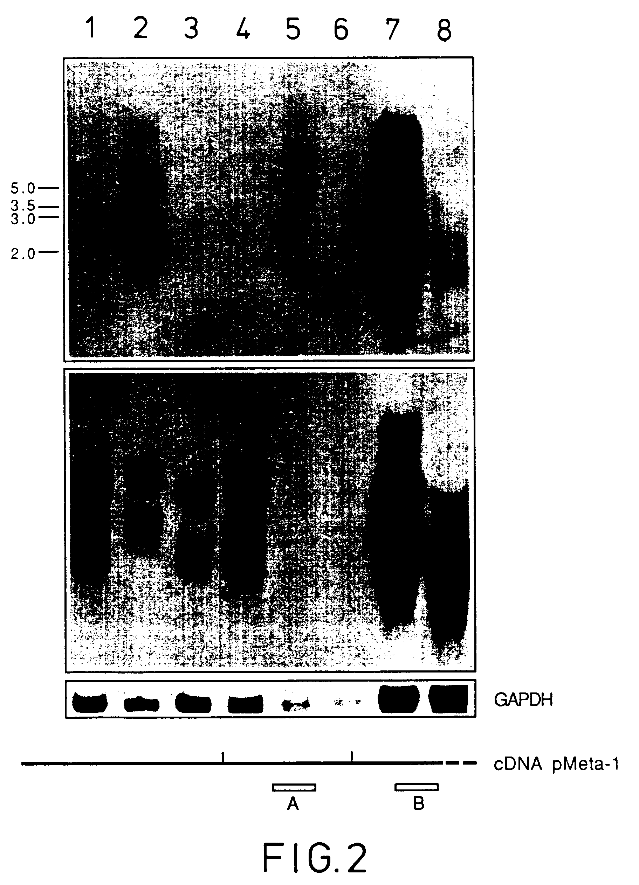

HEIDTMANN et al., Cancer Res. 49, 6960-6965, 1989) wurden jeweils 3µg poly(A)⁺ RNA für die RNA Hybridisierung über Northern Blotting präpariert. Vor dem "Blotting" mit Ethidiumbromid angefärbte RNA im Gel ließ sicherstellen, daß jeweils gleiche Mengen an RNA auf das Gel aufgetragen bzw. pro Bahn vorhanden waren. Die Präparation der poly(A)⁺ RNA, ihre Denaturierung und das Blotting sowie die weiteren Verfahren der RNA Blot Analyse erfolgte nach U. GÜNTHERT et al., 1991, loc. cit.. Die für die Hybridisierung verwendete Probe A aus dem varianten CD44 Bereich von pMeta-1 war identisch mit der wie unter Punkt 1.1. angegeben. Als Probe B, die aus der stromabwärts nach der vCD44 liegenden normalen CD44 Region von pMeta-1 entstammt, wurde der in U. GÜNTHERT et al., 1991, loc. cit. angegebene Bereich ausgewählt. Wie aus der Fig. 2 ersichtlich ist, exprimierte die Human-Zellinie LCLC97 eines großzelligen Lungenkarzinoms Sequenzen, die homolog sind zu der Probe A von vCD44 der Ratte. Hinsichtlich Probe B (normaler sCD44-Bereich der Ratte) waren auch in anderen Zellinien deutliche Hybridisierungssignale bzw. Expressionen von sCD44 nachzuweisen. Die Zellinien SCLC18 und SCLC24 waren für beide Proben negativ. Zum Vergleich wurden die RNAs aus den Rattenzellinien BSpAS (nicht-metastasierend) und BSpASML (metastasierend) aufgetragen (Fig. 2, Bahnen 7 und 8).

### 1.3 Isolierung und Charakterisierung von CD44 Varianten des Menschen

Aus den bekannten Zellinien SW260, HT29 (erhältlich bei der ATCC, Nr. CCL227 bzw. Nr. HTB38), LCLC97 (wie unter 1.2. beschrieben), HPKII (Keratinocyten-Zellinie, beschrieben in P. BOUKAMP et al., J. Cell Biol. 106, 761-771, 1988) und MeWo (Melanoma-Zellinie, T.E. CAREY et al., Proc. Natl. Acad. Sci. USA 73, 3278-3282, 1976) wurden die poly(A)⁺ RNAs nach bekannter Methode isoliert (M. SCHWAB et al., Nature 303, 497-501, 1983) und anschließend für die PCR Amplifikation mit AMV-reverser Trankriptase (20 Einheiten) in einzelsträngige DNA transkribiert.

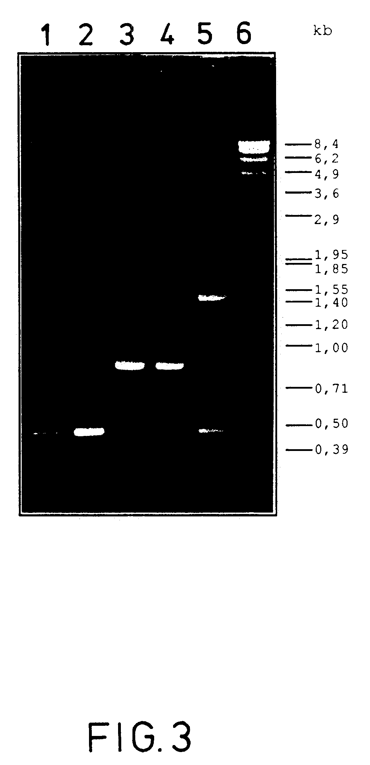

Für die Präparation der cDNAs über PCR Amplifikation wurden zwei Primer aus normalen human CD44 cDNA Sequenzen benutzt und zwar Oligonukleotide, die die Positionen 513 bis 540 und 900 bis 922 repräsentieren (I. STAMENKOVIC et al., Cell 56, 1057-1062, 1989). Diese Auswahl erfolgte daher, da zwischen diesen Positionen die Insertion der metastasen-spezifischen Extrasequenzen bei der Ratte vorgefunden wird (U. GÜNTHERT et al., 1991, loc. cit.). Nach 60 Zyklen wurde die PCR beendet und die erhaltenen DNA Produkte nach bekannter Methode (T. MANIATIS et al., 1982, loc. cit.) über Agarosegelelektrophorese (1% Agarose, Sigma) aufgetrennt und mit Ehtidiumbromid gefärbt. Hinsichtlich der "normalen" CD44 RNA Expression (sCD44) wurde bei Verwendung dieser Primer eine cDNA von 440 bp Länge (einschließlich der Restriktionsstelle für die Klonierung) erhalten, wie sie für die Expression dieser RNA erwartet werden konnte (MeWo und SW620 Zellen). Bei den RNAs aus den anderen Zellinien wird jeweils eine Bande der selben Länge vorgefunden, zusätzlich jedoch größere PCR Produkte (cDNAs). Prominente Fragmente der Länge von 850 bp wurden mit HT29 und LCLC97 Zellen erhalten. In HPKII Zellen war die stärkste Bande, neben jener von 440 bp, im Längenbereich von 1.5 kb aufzufinden. Diese Verhältnisse sind in Fig. 3 dargestellt.

Alle aus den PCR Amplifikationen erhaltenen cDNAs wurden in den Vektor pT7T3-19 (BRL, Gibco) hineinkloniert und anschließend nach Standardmethoden sequenziert. Klone von MeWo Zellen und von SW60 Zellen enthielten die normalen CD44 (sCD44) Sequenzen (I. STAMENKOVIC et al., 1989, loc. cit.).

Die DNA- und Aminosäuresequenz der gesamten varianten Region der längsten aus LCLC97 Zellen über PCR erhaltenen und in pT7T3-19 klonierten cDNA ist in Fig. 4A angegeben. Der variante Teil umfaßt 1014 bp (oder 338 Aminosäuren), welcher zwischen Nukleotidpositionen 782 und 783 der "normalen" sCD44 RNA inseriert ist. Die Sequenz dieses varianten Bereichs ist in fünf Sektionen oder Domänen unterteilt, was in Übereinstimmung ist mit dem Auffinden von kleineren PCR Produkten, welche genau definierte Bereiche des größten Klons (aus LCLC97) umfassen. Aufgrund der Tatsache, daß derartige Klone wiederholbar aus verschiedenen Zellinien isoliert werden konnten, ist abzuleiten, daß diese fünf Domänen D I bis D V der LCLC97 Zellinie (Fig. 4A) fünf unterschiedliche Exons wiederspiegeln, welche, über differentielles RNA-Splicing, die RNAs hervorbringen, von denen die PCR Klone abstammen. Die Existenz derartiger Domänen konnte durch Auffinden gleicher Verhältnisse bei Ratten Tumorzellinien bestätigt werden

Ein schematischer Überblick über die Verhältnisse, wie sie bei LCLC97 vorliegen, zeigt Fig. 4B. Über PCR wurde nach bekannter Methode (U. GÜNTHERT et al., 1991, loc. cit.) ein zum längsten Spleiß-Produkt in LCLC97 Zellen homologer cDNA Klon aus der metastasierenden Ratten Tumorzellinie BSpASML isoliert. Ein Vergleich der davon abgeleiteten Aminosäuresequenz mit derjenigen des menschlichen Klons (aus LCLC97) zeigt Fig. 4A. Die Position der Insertion dieser varianten Sequenzen unterscheidet sich in drei Aminosäuren (Aminosäure Position 222 beim Menschen, Position 226 bei der Ratte). Die Domäne I zeigt 83%, Domäne II 83%, Domäne III 71%, Domäne IV 82% und Domäne V 66% Homologie gegenüber den entsprechenden Aminosäuresequenzen der Ratte.

Die zur LCLC97 in Fig. 4A korrespondierende cDNA der Ratte, welche einem Tumor metastatisches Potential verleiht (U. GÜNTHERT et al., 1991, loc. cit.), umfaßt die Aminosäuren 258 bis 420 und codiert für die Domänen II und III. Die Aminosäuresequenz dieser beiden Domänen ist identisch mit derjenigen von der cDNA des Klons pMeta-1 abgeleiteten Aminosäuresequenz der von U. GÜNTHERT et al., 1991, loc. cit. publizierten BSp73ASML spezifischen Extradomäne.

### Beispiel 2: Immunsuppressive Wirkung des gegen vCD44 gerichteten monoklonalen Antikörpers 1.1 ASML

### 2.1 Einfluß von Anti-vCD44 (1.1ASML) auf die humorale Immunantwort

In BDX Ratten wurden zum Zeitpunkt der Antigenverabreichung 200 µg des monoklonalen Antikörpers 1.1ASML intravenös injiziert. Als T-Zell unabhängiges Antigen wurden 50 µg 2,4,6-Trinitrophenyl-Lipopolysaccharid (TNP-LPS) (J.M. FIDLER, Cellul. Immunol. 16, 223, 1975) und als T-Zell abhängiges antigen 5x10⁸ Zellen des Hapten-Proteinkonjugats 2,4,6-Trinitrophenyl-rote Pferdeblutzellen (TNP-HRBC) (M.B. RITTENBERG, Proc. Soc. Exp. Biol. Med. 132, 575-581, 1969) für die Immunisierung der Ratten intraperitoneal appliziert. Die Anzahl der antigen-spezifischen Plaque bildenden Zellen (plaque forming cells, PFC) wurden 3 und 5 Tage nach der Antigenapplikation bzw. Injektion von 1.1ASML bestimmt, wobei als Zielzellen Konjugate von 2,4,6-Trinitrophenyl mit Schaferythrozyten (sheep red blood cells, TNP-SRBC) und HRBC (Pferdeerythrozyten bzw. horse red blood cells) verwendet wurden. Die PFC wurden nach einer Modifikation (M. ZÖLLER & G. ANDRIGHETTO, Cellul. Immunol. 89, 310, 1984) des hämolytischen Plaque Assays (N.K. JERNE & A.A. NORDIN, Science 140, 405, 1963) bestimmt. Die Mengenbestimmung an Anti-TNP Antikörper im Serum der immunisierten Ratten wurde nach bekannter Methode durchgeführt (M. ZÖLLER, Scand. J. Immunol. 31, 619, 1990), indem Serum Titrationskurven mit Standardkurven von gereinigten Anti-TNP monoklonalen Antikörpern über ELISA (E. ENGVALL & P. PERLMAN, J. Immunol. 109, 129, 1982) verglichen wurden.
Die Immunantworten sowohl gegen das T-Zell unabhängige Antigen als auch gegen das T-Zell abhängige Antigen wurden in Gegenwart von 1.1ASML stark erniedrigt bzw. supprimiert. Die Anzahl antigenspezifischer PFC wurde auf 8% bis 21% gegenüber kontrollstimulierten Tieren reduziert, ebenso wurde der entstandene Serum-Antikörper Spiegel unterdrückt (Tabelle I)

**Tabelle I**

| Einfluß von Anti-vCD44 1.1ASML auf die Aktivierung von B Zellen | | | | |
|---|---|---|---|---|
| Antigen | mAB | PFC/10⁶ SC* | | Serum anti-TNP (mg/ml) |
| | | anti-TNP | anti-HRBC | |
| TNP-LPS | - | 620 | | 108.0 |
| | 1.1ASML | 52 | | 6.1 |
| TNP-HRBC | - | 214 | 1582 | 5.4 |
| | 1.1ASML | 20 | 337 | 2.7 |

| | | | | |
|---|---|---|---|---|
| *SC=Milzzellen | | | | |

Diese Daten erlauben die Interpretation, daß der Antikörper entweder nur über die Hemmung der B-Lymphocytenstimulierung funktioniert oder daß sowohl B- als auch T-Lymphocyten blockiert werden.

Die Expression von vCD44 war jedoch nicht nur für die humorale sondern auch für die zelluläre Immunantwort erforderlich.

### 2.2. Einfluß von Anti-vCD44 auf die Aktivierung von T-Lymphocyten

Die Effizienz einer allogenen Stimulierung wurde 4 Tage nach Immunisierung durch Bestimmung der Proliferation von T-Zellen nach erneuter Stimulierung in vitro ermittelt. Die dafür benutzten DA Ratten wurden entweder nur mit 5x10⁷ bestrahlten Lymphocyten (3000R) von BDX Ratten, oder mit bestrahlten BDX Lymphocyten plus 1.1ASML (200 µg). Ihre Milz- und Lymphknotenzellen wurden mit bestrahlten BDX Lymphocyten in vitro restimuliert. Die Milzen und Lymphknoten wurden 5 Tage später gesammelt. Die Organe wurden vorsichtig zerdrückt und nach Waschen in 15 ml RPMI 1640 wurden die Zellen eingestellt auf 3x10⁶ Zellen/ml RPMI1640, supplementiert mit L-Glutamin (4 mM), Antibiotika (34 µM Penicillin, 32 µM Streptomycin), 5x10⁻⁵M 2-Mercaptoethanol, 10⁻³M HEPES Puffer und 2% hitzeinaktiviertes Rattenserum (RPMI-s). Aliquote der Zellsuspension wurden dreifach in U-förmig vertieften Mikrotiter-Platten titriert. Zu jeder Vertiefung wurden 1.5x10⁵ bestrahlte (3000R) BDX Lymphocyten in 100µl RPMI-s dazugegeben, sowie gegebenenfalls in einer Endkonzentration von 10µg/1ml RPMI-s über Protein A Sepharose® 4B Chromatographie (Pharmacia) gereinigte monoklonale Antikörper 1.1ASML. Die Kulturen wurden für 72h bei 37°C mit 5%iger CO₂ Begasung zur Atmosphäre inkubiert. Die Proliferation der DA Lymphocyten wurde durch Zugabe von 50µCi ³H-Thymidin im Laufe der letzten 8 Stunden der Kultivierung bestimmt.

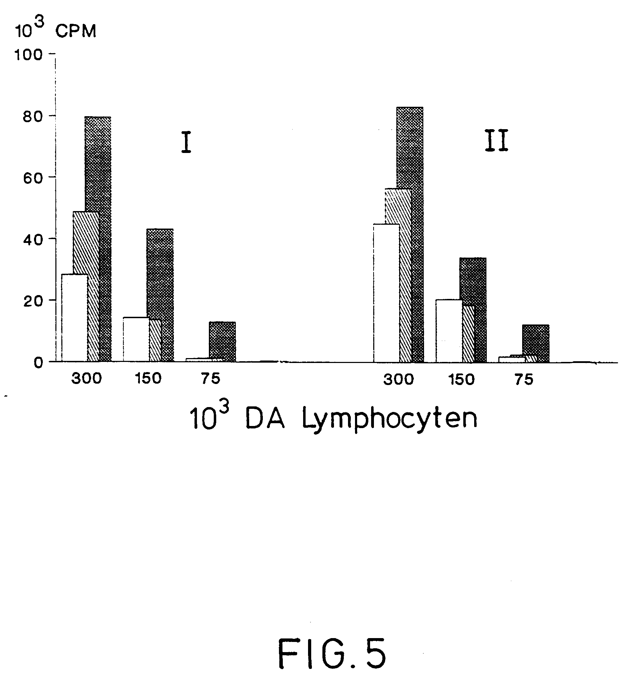

Der Einbau von ³H-Thymidin als Maß für die Proliferation von T-Zellen und Milzzellen von solchen Ratten, die zusammen mit 1.1ASML allogen immunisiert wurden, war, im Vergleich zu Zellen von Ratten, die in Abwesenheit des Antikörpers immunisiert wurden, drastisch reduziert. Dieses Resultat war bestehen geblieben, unabhängig davon, ob Milzzellen und Lymphknotenzellen nur mit allogenen Zellen allein oder mit allogenen Zellen plus 1.1ASML restimuliert wurden (Fig. 5).

### 3.3 Einfluß von Anti-vCD44 auf die Aktivierung von cytotosischen T-Zellen

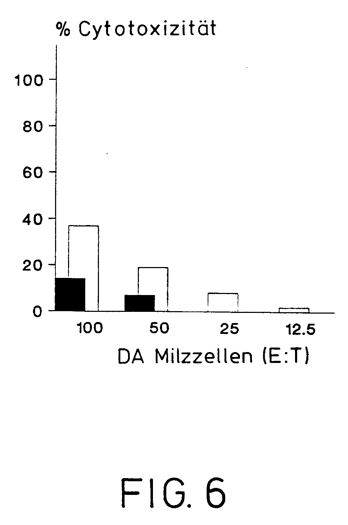

Gegenüber der T-Zell Proliferation in Beispiel 3.2. wurde die cytotoxische Aktivität nach dem siebten Tag der allogenen Stimulation bestimmt. Hinsichtlich der Notwendigkeit von vCD44 während der Reifung und/oder Aktivierung von cytotoxischen T Zellen (CTL) ergab sich das praktisch identische Bild wie unter Beispiel 3.2. (Fig. 5). In Gegenwart von 1.1ASML wurde die Zahl an cytotoxischen T-Lymphocyten drastisch reduziert (Fig. 6).

Milzzellen aus DA Ratten (DA Milzzellen), welche mit BDX Lymphocyten wie unter Beispiel 3.2 beschrieben immunisiert worden waren, wurden 7 Tage nach der Immunisierung gesammelt und auf cytotoxische Aktivität gegen BDX Lymphoblasten getestet (primäre CTL).

Unmittelbar nach Immunisierung gewonnene DA Milzzellen wurden auf 1x10⁷ Zellen/ml RPMI-s eingestellt. Aliquote der Zellsuspension (Effektorzellen, E) wurden in U-förmig vertieften Mikrotiter-Platten titriert und 100µl Zielzellen (Targetcells, T, 1x10⁴ ⁵¹Cr-markierte BDX Concanavalin A Lymphoblasten) wurden dazugegeben. Nach 6stündiger Inkubation bei 37°C wurden die Platten zentrifugiert, Aliquote der Überstände wurden entnommen und die radioaktive Strahlung des ⁵¹Cr in einem γ-Zähler bestimmt. In Fig. 6 ist der gemittelte Prozentsatz der spezifischen Radioaktivität aus drei Werten dargestellt.

## Patentansprüche

1. Verwendung von gegen Metastasen-spezifische Variante von CD44 (vCD44) Oberflächenprotein eines Säugetieres, oder Teilen davon, gerichteten Antikörpern zur Herstellung einer pharmazeutischen Präparation für die Erzeugung einer Immunsuppression in einem Säugetierorganismus.

2. Verwendung der Antikörper nach Anspruch 1 zur Herstellung einer pharmazeutischen Präparation für die Erzeugung einer Immunsuppression zur Verringerung einer Immunantwort.

3. Verwendung nach Ansprüchen 1 oder 2 zur Herstellung einer pharmazeutischen Präparation für die Erzeugung einer Immunsuppression zur therapeutischen Behandlung von immunregulatorischen Störungen und/oder Erkrankungen.

4. Verwendung nach Ansprüchen 1 oder 2 zur Herstellung einer pharmazeutischen Präparation für die Erzeugung einer Immunsuppression zur Prävention und/oder Prophylaxe immunregulatorischer Störungen und/oder Erkrankungen.

5. Verwendung nach Ansprüchen 1 bis 3 zur Herstellung einer pharmazeutischen Präparation für die Erzeugung einer Immunsuppression zur Verhinderung und/oder therapeutischen Behandlung von Krankheiten und Zuständen, bei denen eine vorrübergehende oder dauerhafte Verringerung oder Unterdrückung einer Immunantwort erforderlich ist.

6. Verwendung nach Anspruch 5 zur Herstellung einer pharmazeutischen Präparation für die Erzeugung einer Immunsuppression zur Behandlung von Autoimmunerkrankungen.

7. Verwendung nach Anspruch 6 zur Herstellung einer pharmazeutischen Präparation für die Erzeugung einer Immunsuppression zur Behandlung von allergischen Erkrankungen.

8. Verwendung nach Anspruch 6 zur Herstellung einer pharmazeutischen Präparation für die Erzeugung einer Immunsuppression zur Behandlung von degenerativen, endzündlichen, proliferativen und/oder hyperproliferativen Erkrankungen.

9. Verwendung nach Anspruch 9 zur Herstellung einer pharmazeutischen Präparation für die Erzeugung einer Immunsuppression zur Behandlung von Erkrankungen des rheumatischen Formenkreises, von Multipler Sclerose, Psoriasis oder atopischer Dermatitis.

10. Verwendung nach einem der Ansprüche 1 bis 9 zur Herstellung einer pharmazeutischen Präparation für die Erzeugung einer Immunsuppression zur Verhinderung der Gewebe-und/oder Organabstoßung von transplantierten Geweben oder Organen.

11. Verwendung nach Ansprüchen 1 bis 10, dadurch gekennzeichnet, daß der Säugetierorganismus ein menschlicher Organismus ist.

12. Verwendung nach Ansprüchen 1 bis 11, dadurch gekennzeichnet, daß der Antikörper ein monoklonaler Antikörper ist.

13. Verwendung eines Antikörpers nach einem der Ansprüche 1 bis 12, dadurch gekennzeichnet, daß der Antikörper ein vCD44 Oberflächenprotein oder Teile davon erkennt, welches mindestens als ein zusätzlicher Anteil zur normalen CD44 Domäne (sCD44) vorliegt.

14. Verwendung nach Anspruch 12 oder 13, dadurch gekennzeichnet, daß der monoklonale Antikörper das Epitop, definiert durch die Aminosäuresequenz E-E-A-A-T-Q-K-E-K-W, erkennt.

15. Verwendung nach Ansprüchen 1 bis 14, dadurch gekennzeichnet, daß der Antikörper ein Fragment oder ein Derivat davon ist oder ein chimärer oder Hybridantikörper, ein antiidiotypischer Antikörper oder ein bispezifischer Antikörper ist.

16. Verwendung nach einem der Ansprüche 1 bis 15, dadurch gekennzeichnet, daß die den Antikörper enthaltende pharmazeutische Präparation als gebrauchsfertige Lösung, als Trockenpräparat in einer für die Rekonstitution geeigneten Form oder als Kit vorliegt.

17. Verwendung nach Anspruch 16, dadurch gekennzeichnet, daß die den Antikörper enthaltende pharmazeutische Präparation in einer enteral oder parenteral, systemisch, lokal und/oder topisch zu verabreichenden Applikationsform vorliegt.

18. Verwendung nach Ansprüchen 16 und 17, dadurch gekennzeichnet, daß die den Antikörper enthaltende pharmazeutische Präparation in einer für die Injektion, Infusion oder Perfusion geeigneten Darreichungsform vorliegt.

## Claims

1. Use of antibodies directed against metastasis-specific variants of CD44 (vCD44) surface protein of a mammal, or parts thereof, in the manufacture of a pharmaceutical preparation for producing immunosuppression in a mammalian organism.

2. Use of the antibodies according to claim 1 for manufacturing a pharmaceutical preparation for producing immunosuppression in order to reduce an immune response.

3. Use according to claim 1 or 2 for manufacturing a pharmaceutical preparation for producing immunosuppression for the therapeutic treatment of immunoregulatory disorders and/or diseases.

4. Use according to claim 1 or 2 for manufacturing a pharmaceutical preparation for producing immunosuppression for the prevention and/or prophylaxis of immunoregulatory disorders and/or diseases.

5. Use according to claims 1 to 3 for manufacturing a pharmaceutical preparation for producing immunosuppression for the prevention and/or therapeutic treatment of diseases and conditions in which a temporary or lasting reduction or suppression of an immune response is required.

6. Use according to claim 5 for manufacturing a pharmaceutical preparation for producing immunosuppression for treating autoimmune diseases.

7. Use according to claim 6 for manufacturing a pharmaceutical preparation for producing immunosuppression for treating allergic diseases.

8. Use according to claim 6 for manufacturing a pharmaceutical preparation for producing immunosuppression for treating degenerative, inflammatory, proliferative and/or hyperproliferative diseases.

9. Use according to claim 9 for manufacturing a pharmaceutical preparation for producing immunosuppression for treating diseases of the rheumatic type, multiple sclerosis, psoriasis or atopic dermatitis.

10. Use according to claims 1 to 9 for manufacturing a pharmaceutical preparation for producing immunosuppression for preventing tissue and/or organ rejection in the transplanting of tissues or organs.

11. Use according to claims 1 to 10, characterised in that the mammalian organism is a human body.

12. Use according to claims 1 to 11, characterised in that the antibody is a monoclonal antibody.

13. Use of an antibody according to one of claims 1 to 12, characterised in that the antibody recognises a vCD44 surface protein or parts thereof, which is present at least as an additional component to the normal CD44 domain (sCD44).

14. Use according to claim 12 or 13, characterised in that the monoclonal antibody recognises the epitope defined by the amino acid sequence E-E-A-A-T-Q-K-E-K-W.

15. Use according to claims 1 to 14, characterised in that the antibody is a fragment or a derivative thereof or a chimeric or hybrid antibody, an anti-idiotypic antibody or a bispecific antibody.

16. Use according to one of claims 1 to 15, characterised in that the pharmaceutical preparation containing the antibody is present as a ready-to-use solution, as a dry preparation in a form suitable for reconstitution or as a kit.

17. Use according to claim 16, characterised in that the pharmaceutical preparation containing the antibody is in a form for administration by enteral or parenteral, systemic, local and/or topical route.

18. Use according to claims 16 and 17, characterised in that the pharmaceutical preparation containing the antibody is present in a form suitable for injection, infusion or perfusion.

## Revendications

1. Utilisation d'anticorps dirigés contre une variante spécifique de métastases de la protéine de surface CD44 (vCD44) d'un mammifère, ou de parties de celle-ci, pour la production d'une préparation pharmaceutique pour l'obtention d'une immunosuppression dans un organisme de mammifère.

2. Utilisation des anticorps selon la revendication 1 pour la production d'une préparation pharmaceutique pour l'obtention d'une immunosuppression pour réduire une réponse immunitaire.

3. Utilisation selon la revendication 1 ou 2 pour la production d'une préparation pharmaceutique pour l'obtention d'une immunosuppression pour le traitement thérapeutique de troubles et/ou maladies de l'immunorégulation.

4. Utilisation selon la revendication 1 ou 2 pour la production d'une préparation pharmaceutique pour l'obtention d'une immunosuppression pour la prévention et/ou la prophylaxie de troubles et/ou maladies de l'immunorégulation.

5. Utilisation selon les revendications 1 à 3 pour la production d'une préparation pharmaceutique pour l'obtention d'une immunosuppression pour empêcher et/ou traiter thérapeutiquement des maladies et des états dans lesquels une réduction ou répression provisoire ou durable d'une réponse immunitaire est nécessaire.

6. Utilisation selon la revendication 5 pour la production d'une préparation pharmaceutique pour l'obtention d'une immunosuppression pour le traitement de maladies auto-immunes.

7. Utilisation selon la revendication 6 pour la production d'une préparation pharmaceutique pour l'obtention d'une immunosuppression pour le traitement de maladies allergiques.

8. Utilisation selon la revendication 6 pour la production d'une préparation pharmaceutique pour l'obtention d'une immunosuppression pour le traitement de maladies dégénératives, inflammatoires, prolifératives et/ou hyperprolifératives.

9. Utilisation selon la revendication 9 pour la production d'une préparation pharmaceutique pour l'obtention d'une immunosuppression pour le traitement de maladies du domaine des formes rhumatismales, de la sclérose en plaques, du psoriasis ou de la dermatite atopique.

10. Utilisation selon l'une des revendications 1 à 9 pour la production d'une préparation pharmaceutique pour l'obtentlon d'une immunosuppression pour empêcher le rejet de tissu et/ou d'organe de tissus ou d'organes transplantés.

11. Utilisation selon les revendications 1 à 10 caractérisée en ce que l'organisme de mammifère est un organisme humain.

12. Utilisation selon les revendications 1 à 11 caractérisée en ce que l'anticorps est un anticorps monoclonal.

13. Utilisation d'un anticorps selon l'une des revendications 1 à 12 caractérisée en ce que l'anticorps reconnaît une protéine de surface vCD44, ou des parties de celle-ci, qui est présente au moins sous forme d'une partie supplémentaire du domaine de CD44 normal (sCD44).

14. Utilisation selon la revendication 12 ou 13 caractérisée en ce que l'anticorps monoclonal reconnaît l'épitope défini par la séquence d'acides aminés E-E-A-A-T-Q-K-E-K-W.

15. Utilisation selon les revendications 1 à 14 caractérisée en ce que l'anticorps est un fragment ou dérivé d'anticorps ou est un anticorps chimérique ou hybride, un anticorps anti-idiotypique ou un anticorps bispécifique.

16. Utilisation selon l'une des revendications 1 à 15 caractérisée en ce que la préparation pharmaceutique contenant l'anticorps est sous forme de solution prête à l'emploi, sous forme de préparation sèche dans une forme appropriée à la reconstitution ou sous forme de trousse.

17. Utilisation selon la revendication 16 caractérisée en ce que la préparation pharmaceutique contenant l'anticorps est présente dans une forme d'application à administrer par voie entérale ou parentérale, systémique, locale et/ou topique.

18. Utilisation selon les revendications 16 et 17 caractérisée en ce que la préparation pharmaceutique contenant l'anticorps est présente dans une forme d'administration appropriée à l'injection, l'infusion ou la perfusion.
